(19) Europäisches Patentamt
European Patent Office
Office européen des brevets

(11) EP 3 090 635 A1

(12) EUROPEAN PATENT APPLICATION
published in accordance with Art. 153(4) EPC

(43) Date of publication:
09.11.2016 Bulletin 2016/45

(51) Int Cl.:
A23L 2/38 (2006.01)        A23C 9/00 (2006.01)
A23L 2/52 (2006.01)

(21) Application number: 14875902.0

(22) Date of filing: 11.07.2014

(86) International application number:
PCT/KR2014/006253

(87) International publication number:
WO 2015/102188 (09.07.2015 Gazette 2015/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 31.12.2013 KR 20130169402

(71) Applicant: LOTTE Fine Chemical Co., Ltd.
Ulsan, 44714 (KR)

(72) Inventors:
• CHA, Ja Hyun
Incheon 405-734 (KR)

• LEE, Hyun Woo
Incheon 404-310 (KR)
• LEE, Eun Jung
Seoul 137-797 (KR)
• HONG, Jun Kee
Yongin-si
Gyeonggi-do 448-728 (KR)
• LEE, Sung Wan
Incheon 402-803 (KR)
• KO, Won Hwa
Incheon 403-862 (KR)

(74) Representative: Kador & Partner
Corneliusstraße 15
80469 München (DE)

(54) **FUNCTIONAL BEVERAGE**

(57) A functional beverage is disclosed. The disclosed functional beverage comprises a mother liquor and a liquid additive, wherein the liquid additive comprises a poorly water-soluble natural product, an edible surfactant, an edible cosurfactant, and edible oil.

FIG. 1

**Description**

TECHNICAL FIELD

The inventive concept relates to a functional beverage, and more particularly, to a functional beverage with excellent in-vivo absorption rate and bioavailability.

BACKGROUND ART

Beverages have a primary function of supplying nutrition; a secondary function of providing joyful feeling by taste, aroma, and physical properties; and a tertiary function of contributing in disease prevention and health improvement due to a body modulating function provided by various bioactive components contained in the beverage.

Internationally, including in the Republic of Korea, health functional beverage markets have been focused on preventing diseases and improving health through a body modulating function of a beverage as the population ages. Also, there is tendency that the health functional beverage markets are rapidly grown. In this regard, many food companies have actively conducted discoveries of various materials and beverage development using the discovered materials.

Among natural products, many are proved for its safety through the long time human history, unlike general compounds. Development of food using such substances has also widely conducted. In this case, in-vivo absorption rate and bioavailability are the most important factors. However, in the field of beverages, unlike in the field of pharmaceutics, development of techniques to improve in-vivo absorption rate and bioavailability of the functional material having a physiological function is yet insignificant.

DETAILED DESCRIPTION OF THE INVENTIVE CONCEPT

TECHNICAL PROBLEM

The inventive concept provides a functional beverage with excellent in-vivo absorption rate and bioavailability.

TECHNICAL SOLUTION

According to an aspect of the inventive concept, there is provided a functional beverage including a mother liquor; and a liquid additive, wherein the liquid additive comprises a poorly water-soluble natural product, an edible surfactant, an edible cosurfactant, and an edible oil.

The mother liquor may be whole milk, coffee milk, soy milk, fermented milk, low-fat milk, fat-free milk, functional milk, or recombined milk.

The liquid additive may exist in a state of emulsion that is dispersed in the mother liquor.

An amount of the liquid additive may be in a range of 0.5 parts to 10 parts by weight based on 100 parts by weight of the mother liquor.

The liquid additive may include 100 parts by weight of the poorly water-soluble natural product, about 300 parts to about 8,000 parts by weight of the edible surfactant, about 40 parts to about 2,500 parts by weight of the edible cosurfactant, and about 40 parts to about 1,500 parts by weight of the edible oil.

The poorly water-soluble natural product may include at least one type of a poorly water-soluble natural polyphenol compound selected from curcumin, silymarin, and resveratol.

The edible surfactant may include polysorbates.

The edible cosurfactant may include at least one selected from glycerol fatty acid esters, propylene glycols, propyleneglycol fatty acid esters, and medium chain triglycerides.

The edible oil may include at least one selected from vegetable oil, refined fish oil, and oils derived from seaweed.

The liquid additive further may include an edible additive at a ratio of about 500 parts by weight or lower.

The edible additive may include at least one compound selected from lecithin, a viscosity controlling agent, flavoring agent, preservatives, colorant, glycerol, sorbitol, and gelatin.

ADVANTAGEOUS EFFECTS OF THE INVENTION

According to an embodiment of the inventive concept, provided is a functional beverage with excellent in-vivo absorption rate and bioavailability.

DESCRIPTION OF THE DRAWINGS

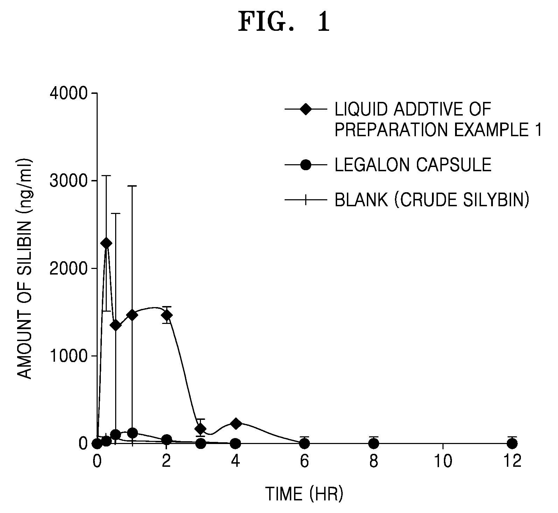

FIG. 1 is a graph illustrating a PK test data of a liquid additive prepared in Preparation Example 1 along with a PK test data of a commercial liquid additive and blank.

MODE OF THE INVENTIVE CONCEPT

Hereinafter, a functional beverage according to an embodiment of the inventive concept will be described.

The functional beverage according to an embodiment of the inventive concept includes a mother liquor and a liquid additive.

As used herein, the term "functional beverage" denotes a beverage that is absorbed in vivo and includes a bioactive component having a physiological function.

Also, as used herein, the term "mother liquor" denotes a part of the functional beverage other than the liquid additive, and the term "liquid additive" denotes a part that gives functionality to the functional beverage and increases in-vivo absorption rate and bioavailability of the functional beverage.

The mother liquor may be whole milk, coffee milk, soymilk, fermented milk, low-fat milk, fat-free milk, functional milk, or recombined milk.

The whole milk denotes raw milk, from which fat is not removed at all, that undergoes commercial sterilization for several seconds at a temperature in a range of 120°C to 140°C or undergoes low-temperature sterilization (e.g., for 30 minutes at 63°C or 15 seconds at 72°C). The whole milk may include milk fat at an amount of 3 wt% or more.

The raw milk refers to non-processed milk squeezed from milk cows.

The coffee milk may include raw milk, coffee, and water. Also, the coffee milk may further include at least one compound selected from sugar, milk cream, sodium hydrogen carbonate, coffee flavor, and sucrose fatty acid ester. The coffee milk may be prepared by mixing raw milk and water and heating the resultant at a temperature in a range of 65°C to 70°C to obtain diluted milk, mixing pre-mix coffee and the diluted milk to obtain a mixture solution, and adding milk cream and coffee flavor to the mixture solution. The pre-mix coffee may include at least two compounds selected from coffee powder, sugar, and sucrose fatty acid ester. The coffee milk may include milk fat at an amount in a range of 1 wt% to 3 wt%.

The soymilk may include a soymilk solution, seasoning, and water.

In the soymilk, an amount of water may be in a range of 85 wt% to 90 wt%.

The soymilk solution may be a milky liquid extracted from soybeans and include at least 7 wt% of soybean solid.

The seasoning may include at least one selected from vegetable oil, saccharides, and table salt. For example, the seasoning may include at least one selected from refined sugar, mixed grain powder, black bean extract, black sesame paste, refined salt, glycerin ester of fatty acid, and flavoring agents.

The mixed grain powder may include at least two grains selected from wheat, barley, mung beans, and brown rice.

The flavoring agent may include at least one selected from coffee flavor, milk flavor, cream flavor, vanilla flavor, butter flavor, coconut flavor, chocolate flavor, oleaginous seed flavor, green tea flavor, sweet potato flavor, black tea flavor, blueberry flavor, and sweet pumpkin flavor.

The fermented milk may be stirred-type fermented milk or set-type fermented milk. The stirred-type fermented milk is prepared by putting commercialized fermentation bacteria into a large-sized fermentation tub filled with pre-sterilized milk and culturing the fermentation bacteria; determining a culturing completion point of time to terminate the culturing of the fermentation bacteria by measuring pH or acidity of the content of the fermentation tub; terminating the culturing of the fermentation bacteria; and additionally adding other raw materials such as syrup into the fermentation tub and mixing the resultant.

The set-type fermentation milk is also referred to as homemade type yogurt, which is released as a product after containing the whole fermentation base including fermentation bacteria (i.e., lactobacteria) in a small container and packing the container, fermenting the fermentation base in the container in a fermentation chamber, and putting the container into cold storage.

The low-fat milk refers to milk prepared by partially removing a milk fat component from raw milk, wherein an amount of the milk fat component of low-fat milk may be 1/2 to 1/100 of an amount of the milk fat component of raw milk.

The fat-free milk refers to milk prepared by removing all the milk fat components from raw milk.

The functional milk may refer to milk with enhanced functionality by adding various functional components, such as calcium or docosa hexaenoic acid (DHA), to the whole milk.

The recombined milk refers to milk prepared by dissolving at least one selected from whole milk powder, skimmed milk powder, and whey powder in water. Examples of the recombined milk may include recombined whole milk, recombined low-fat milk or a combination thereof. The recombined milk may be one prepared by additional pasteurization and sterilization process. When preparing the recombined milk, amounts of the whole milk powder and skimmed milk powder may be appropriately selected. For example, 12 wt% of the whole milk powder may be used in preparation of the recombined whole milk (that is, dissolving 12 g of the whole milk powder in 88 g of water), and 10 wt% of the skimmed milk powder may be used in preparation of the recombined low-fat milk (that is, dissolving 10 g of the skimmed milk powder in 90 g water).

Unlike milk in the liquid state, the recombined milk uses milk powder which is in the powder state during storage and distribution, and thus the recombined milk is advantageous in terms of its expiration date and ease of handling compared to those of milk in the liquid state. However, the recombined milk is heat-treated at a higher temperature, and thus a denaturation degree of protein is generally severe compared to that of liquid milk product, so that the recombined milk may have a significantly low foam forming ability. For example, uniformity foam may not be formed when the whole milk powder is used, and although thick foam may be formed in a case of using the skimmed milk powder, uniformity and quality of the foam in the case are inferior to the case of using the whole milk.

The liquid additive includes a poorly water-soluble natural product, an edible surfactant, an edible cosurfactant, and an edible oil.

The liquid additive may exist in the state of emulsion that is dispersed in the mother liquor. That is, the functional beverage may include the mother liquor and emulsion of the liquid additive dispersed in the mother liquor. In this regard, when a consumer drinks the functional beverage, the emulsion of the liquid additive in-flows into the body with the mother liquor, and since the poorly water-soluble natural product contained in the emulsion of the liquid additive exists in the state dissolved in the liquid additive, the liquid additive has a high bioavailability due to a high in-vivo absorption rate. When the poorly water-soluble natural product alone is added to the mother liquor, instead of being in the state dissolved in the liquid additive, a solubility of the poorly water-soluble natural product with respect to the mother liquor is low, and thus a bioavailability is low due to a low in-vivo absorption rate (see Evaluation Example 3).

The liquid additive may be a mixture, i.e., a solution, in which all components including the poorly water-soluble natural product are homogenously mixed therein. In this regard, when a consumer drinks the functional beverage, the liquid additive increases an in-vivo absorption rate of the poorly water-soluble natural product and thus improves the bioavailability thereof.

An amount of the liquid additive may be in a range of 0.5 parts to 10 parts by weight based on 100 parts by weight of the mother liquor. However, embodiments of the inventive concept are not limited thereto, and the amount of the liquid additive may variously change according to types and compositions of the mother liquor.

The liquid additive may include the poorly water-soluble natural product at an amount of 100 parts by weight, the edible surfactant at an amount in a range of 300 parts to 8,000 parts by weight, the edible cosurfactant at an amount in a range of 40 parts to 2,500 parts by weight, and the edible oil at an amount in a range of 40 parts to 1,500 parts by weight. As used herein, the term "edible" refers to a subject that is sitologically permissible.

The poorly water-soluble natural product may have health promoting function and/or physiological function.

The poorly water-soluble natural product may include a poorly water-soluble natural polyphenol compound. For example, the poorly water-soluble natural polyphenol compound may include curcumin, silymarin, resveratrol, or a combination thereof. The curcumin may be derived from a turmeric root extract. The silymarin may be derived from a milk thistle extract. Also, the silymarin may include silybin, isosilybin, silychristin, and silydianin.

The edible surfactant increases solubility of the poorly water-soluble natural product in the liquid additive and dispersibility of the liquid additive in the mother liquor.

The edible surfactant may include polysorbates. The polysorbates may include polysorbate 20, polysorbate 40, polysorbate 60, polysorbate 80, or a combination thereof.

When the amount of the edible surfactant is within this range based on 100 parts by weight of the poorly water-soluble natural product, the poorly water-soluble natural product may be sufficiently dissolved in the liquid additive, and the liquid additive may form an emulsion in the mother liquor.

The edible cosurfactant additionally increases solubility of the poorly water-soluble natural product in the liquid additive and dispersibility of the liquid additive in the mother liquor.

The edible cosurfactant may include at least one compound selected from glycerol fatty acid esters, propylene glycols, and propylene glycol fatty acid esters. The glycerol fatty acid esters may include glycerol dibehenate, glycerol monooleate, polyglycerol oleate, glycerol palmitostearate, or a combination thereof. The propylene glycol fatty acid esters may include propylene glycol monocaprylate, propylene glycol dicaprylocaprate, propylene glycol laurate, or a combination thereof. For example, the edible cosurfactant may include Plurol®Oleique, Capryl™ PGMC, propylene glycol, or a combination thereof.

When an amount of the edible cosurfactant is within this range based on 100 parts by weight of the poorly water-soluble natural product, the liquid additive may form an emulsion in the mother liquor, and the small-sized emulsion particles may be formed, which results in an increase in in-vivo absorption rate and improvement in bioavailability.

When glycerol fatty acid ester is used as the edible cosurfactant, an amount of the glycerol fatty acid ester may be in a range of 40 parts to 1,500 parts by weight, or, for example, 60 parts to 1,000 parts by weight, based on 100 parts by weight of the poorly water-soluble natural product. Also, when propylene glycol fatty acid ester is used as the edible cosurfactant, an amount of the propylene glycol fatty acid ester may be in a range of 100 parts to 2,500 parts by weight, or, for example, 120 parts to 2,000 parts by weight, based on 100 parts by weight of the poorly water-soluble natural product.

The edible oil additionally increases solubility of the poorly water-soluble natural product in the liquid additive and dispersibility of the liquid additive in the mother liquor. Also, the edible oil may have a physiological function.

The edible oil may include at least one selected from vegetable oil, refined fish oil, and oils derived from seaweed. For example, the edible oil may include olive oil, medium chain triglyceride (MCT) oil, fish oil, Ahi flower oil, Algae oil, or a combination thereof. The MCT oil may be refined processed fat and oil as one type of edible fat and oil.

When the amount of the edible oil is within this range based on 100 parts by weight of the poorly water-soluble natural product, the liquid additive may form an emulsion in the mother liquor.

The liquid additive may further include an edible additive at a ratio in a range of greater than 0 part to 500 parts by weight based on 100 parts by weight of the poorly water-soluble natural product.

The edible additive may include at least one selected from lecithin, a viscosity controlling agent, flavoring agent, preservative, colorant, glycerol, sorbitol, and gelatin.

The viscosity controlling agent may include a water-soluble polymer such as polyvinylpyrrolidone to increase dispersibility and absorptivity of the solid.

The flavoring agent may include an ether, an ester, a ketone, a fatty acid, a phenol, an aromatic alcohol, or a combination thereof. For example, the flavoring agent may include geranyl formate, citronellyl formate, isoamyl formate, cinnamic acid, or a combination thereof.

The preservative may include a dehydroacetic acid, a sorbic acid, a benzoic acid, a propionic acid, or a combination thereof. For example, the preservative may include a synthetic additive such as dibutylhydroxytoluene or butylhydroxyanisole; a natural additive such as D-tocopherol or a defatted ricebran extract; or a combination thereof.

The colorant may include a natural colorant such as turmeric, saffron, or green cholophyll; an artificial colorant designated in a food additive list such as edible colorant Green No. 3, edible colorant Red No. 3, or edible colorant Yellow No. 5; or a combination thereof.

The liquid additive exists in the state of an emulsion in the mother liquor, and thus, when a consumer drinks the functional beverage, active ingredients in the poorly water-soluble natural product are absorbed into the body within several minutes. Thus, the liquid additive may maximize the bioavailability of the poorly water-soluble natural product.

Hereinafter, one or more embodiments of the inventive concept will be described in detail with reference to the following examples. However, these examples are not intended to limit the scope of the one or more embodiments of the inventive concept.

Example

Preparation Example 1: Preparation of liquid additive

First, an edible surfactant, an edible cosurfactant, and an edible oil were homogenously mixed at a ratio shown in Table 1. Then, the poorly water-soluble natural product was added to the resultant at a ratio shown in Table 1 and the resultant was homogenously mixed to sufficiently dissolve the poorly water-soluble natural product, and thus a liquid additive was prepared. However, in Table 1, silymarin was available from NATUREX in France, polysorbate 80 was EMASOL O-120V available from KAO Chemical in Japan, Plurol®Oleique is a brand name of polyglyceryl-3 dioleate available from Gattefosse Co. in France, and olive oil was available from Borges extra virgin in Spain.

[Table 1]

| Substance | Component | Amount (part by weight) |
|---|---|---|
| Poorly water-soluble natural product | Silymarin | 10.7 |
| Edible surfactant | Polysorbate 80 | 40.2 |
| Edible cosurfactant | Plurol®Oleique | 46.9 |
| Edible oil | Olive oil | 2.2 |
| Total | | 100 |

Example 1: Preparation of functional whole milk

First, whole milk (including solid at an amount of 12 wt% available from Maeil Dairies Co., Ltd.) was heated to a temperature in a range of 65°C to 70°C. Then, 98.8 parts by weight of the heated whole milk and 1.2 parts by weight of the liquid additive prepared in Preparation Example 1 were mixed to obtain a first mixture, and the first mixture was mixed at a stirring rate of about 3,000 rpm for 3 minutes to 5 minutes to disperse the liquid additive well in the whole milk. As a result, a second mixture was obtained. Subsequently, the second mixture was homogenized at 180 bar to 200 bar by using a homogenizer for beverage (Panda Plus 1000 available from Niro Soavi), sterilized at 135°C for 2 seconds to 3 seconds, and cooled to a temperature of 5°C. As a result, functional whole milk was obtained.

Example 2 and Comparative Example 1: Preparations of functional coffee milk and non-functional coffee milk

First, raw milk (including solid at an amount of 12 wt% available from Maeil Dairies Co., Ltd.) and purified water were mixed and the resultant was heated to a temperature in a range of 65°C to 70°C. As a result, a first mixture was obtained. Then, a mixture powder including premixed sugar (Beksul white sugar, available from CJ), coffee powder (Maxim Arabica, available from Dongsuh Food), and sodium hydrogen carbonate (available from ES Food Raw Material) were added to the first mixture. As a result, a second mixture was obtained. Then, milk cream (Seoul Milk fresh cream, available from Seoul Milk), the liquid additive prepared in Preparation Example 1, and a flavoring agent (coffee flavor, available from Givaudan) were added to the second mixture in this stated order to obtain a third mixture, and the third mixture was mixed at a stirring rate of about 3,000 rpm for 3 minutes to 5 minutes to disperse the components in the purified water. As a result, a fourth mixture was obtained. Subsequently, the fourth mixture was homogenized at 180 bar to 200 bar by using a homogenizer for beverage (Panda Plus 1000, available from Niro Soavi), sterilized at 135°C for 2 seconds to 3 seconds, and cooled to 5°C. As a result, a functional coffee milk (Example 2) and a non-functional coffee milk (Comparative Example 1) were obtained. Composition of each of the coffee milks is shown in Table 2. However, values shown in Table 2 are amounts, and the unit of the amounts is part by weight.

[Table 2]

| Component | Example 2 | Comparative Example 1 |
|---|---|---|
| Raw milk | 60 | 60 |
| Sugar | 4 | 4 |
| Coffee powder | 0.6 | 0.6 |
| Milk cream | 0.8 | 0.8 |
| Sodium hydrogen carbonate | 0.1 | 0.1 |
| Flavoring agent | 0.08 | 0.05 |
| Liquid additive prepared in Preparation Example 1 | 1 | 0 |
| Purified water | 33.42 | 34.45 |
| Total | 100 | 100 |

Example 3 and Comparative Example 2: Preparations of functional soymilk and non-functional soymilk

First, a soymilk solution (including soybean solid at an amount of 7 wt%, pH: 7.0±0.3, available from Yonsei Milk) was heated to a temperature in a range of 65°C to 70°C. Then, refined white sugar (Beksul white sugar, available from CJ), a mixed grain powder (7-grains mixed powder, available from Keonwu F/P), refined salt (Hanju, available from Hanju Salt), and glycerin fatty acid ester (IlshinWellga) were premixed and added to the heated soymilk solution. As a result, a first mixture was obtained. Then, a black bean extract (MSC), a black sesame paste (Jinsung F/M), the liquid additive prepared in Preparation Example 1, a flavoring agent (soybean flavor, available from Givaudan), and purified water were added to the first mixture. As a result, a second mixture was obtained. Then, the second mixture was mixed at a stirring rate of about 3,000 rpm for 5 minutes to disperse the components in the purified water. As a result, a third mixture was obtained. Subsequently, the third mixture was homogenized at 250 bar to 300 bar by using a homogenizer for beverage (Panda Plus 1000, available from Niro Soavi), sterilized at 135°C for 15 seconds to 30 seconds, and cooled to 5°C. As a result, a functional soymilk (Example 3) and a non-functional coffee milk (Comparative Example 2) were obtained. Composition of each of the soymilks is shown in Table 3. However, values shown in Table 3 are amounts, and the unit of the amounts is part by weight.

[Table 3]

| Component | Example 3 | Comparative Example 2 |
|---|---|---|
| Soymilk solution | 90 | 90 |
| Refined white sugar | 4 | 4 |
| Mixed grain powder | 1.1 | 1.1 |
| Black bean extract | 0.5 | 0.5 |
| Black sesame paste | 0.5 | 0.5 |
| Refined salt | 0.15 | 0.15 |
| Glycerin fatty acid ester | 0.1 | 0.1 |
| Flavoring agent | 0.1 | 0.05 |
| Liquid additive prepared in Preparation Example 1 | 1 | 0 |
| Purified water | 2.55 | 3.6 |
| Total | 100 | 100 |

Example 4 and Comparative Example 3: Preparations of functional fermented milk and non-functional fermented milk

First, raw milk (including solid at an amount of 12 wt% available from Maeil Dairies Co., Ltd.) was heated to a temperature in a range of 65°C to 70°C. Then, a skimmed milk powder (Seoul Milk) was added to the heated raw milk and the resultant was mixed at a stirring rate of about 3,000 rpm for 3 minutes. As a result, a first mixture was obtained. Subsequently, the first mixture was sterilized at 83±3°C for 15 minutes to 20 minutes, and cooled to 40±2°C. As a result, a second mixture was obtained. Subsequently, an ABT-5 mixture strain (available from Christian Hansen) was added to the second mixture, and the strain was cultured at a temperature in a range of 40°C to 44°C for 6.5 hours. Here, an adding amount of the ABT-5 mixture strain was 0.01 sales unit (SU) based on 100 g of the second mixture. As a result, a third mixture (also, referred to as 'a culture solution') was obtained. A pH of the culture solution was 4.3±0.05, and an acidity was 0.75±0.05. Then, purified water was heated to a temperature in a range of 65°C to 70°C. Then, sugar (Beksul white sugar, available from CJ), a mixed grain powder (7-grains mixed powder, available from Keonwu F/P), and HPMC (AnyAddy®HPMC 2208, available from Samsung Fine Chemicals Co., Ltd.) were premixed and added to the heated purified water. As a result, a fourth mixture was obtained. Then, the liquid additive prepared in Preparation Example 1 was added to the fourth mixture, and the resultant was mixed at a stirring rate of about 3,000 rpm for 5 minutes. As a result, a fifth mixture was obtained. Then, the fifth mixture was sterilized at 83±3°C for 15 minutes to 20 minutes and cooled to 42°C. As a result, a sixth mixture (also, referred to as 'a syrup solution') was obtained. Subsequently, the culture solution and the syrup solution were well-mixed. As a result, a seventh mixture was obtained. Thereafter, a flavoring agent (yogurt flavor, available from Givaudan) was added to the seventh mixture. As a result, an eighth mixture was obtained. Then, the eighth mixture was homogenized at 180 bar by using a homogenizer for beverage (Panda Plus 1000, available from Niro Soavi) and cooled to 5°C. As a result, a functional fermented milk (Example 4) and a non-functional fermented milk (Comparative Example 3) were obtained. Composition of each of the fermented milks is shown in Table 4. However, values shown in Table 4 are amounts, and the unit of the amounts is part by weight.

[Table 4]

| Component | Example 4 | Comparative Example 3 |
|---|---|---|
| Raw milk | 80 | 80 |
| Skimmed milk powder | 2.5 | 2.5 |
| Sugar | 6 | 6 |
| Grain powder | 1 | 1 |
| Liquid additive prepared in Preparation Example 1 | 1.5 | 0 |
| HPMC | 0.15 | 0.15 |
| Flavoring agent | 0.15 | 0.1 |
| Purified water | 8.7 | 10.25 |

(continued)

| Component | Example 4 | Comparative Example 3 |
|---|---|---|
| Total | 100 | 100 |

Evaluation Example

Evaluation Example 1: Evaluation of appearance of liquid additive

The liquid additive prepared in Preparation Example 1 was left out overnight, and the appearance thereof was observed with the naked eyes. As the result, layer separation did not occur in the liquid additive prepared in Preparation Example 1 at all, and thus it was confirmed that the liquid additive was a solution, in which four components were homogenously mixed. In this regard, when a consumer drinks a functional beverage including the liquid additive, it may be easily expected that in-vivo absorption rate and bioavailability of the poorly water-soluble natural product contained in the liquid additive may be excellent.

Evaluation Example 2: Pharmacokinetic (PK) test

The liquid additive prepared in Preparation Example 1 or a commercial liquid additive is orally administrated to Sprague-Dawley male rats, and in-vivo absorption rates of each of the liquid additives in the Sprague-Dawley male rats were evaluated, and the results are shown in Table 5 and FIG. 1. In particular, six rats (at a body weight in a range of 200 g to 300 g and an age of about 9 weeks) per one type of the liquid additives were used. In particular, while the rats were raised in a cage under the same condition for 8 days, a predetermined amount of common solid forage and water were provided so that the rats would freely take food and water. Then, the rats were fasted for 16 hours, and the PK test was performed by using each of the liquid additives. Specifically, a predetermined amount of silymarin (i.e., an amount of silymarin to administrate 66.67 mg of silybin per 1 kg of the rat's body weight) was once administrated with water to each of the rats by means of forced oral administration using an oral administration device. After about 4 hours from orally administrating each of the liquid additives to each of the rats, the solid forage and water were provided again. Blood was directly obtained from jugular vein of each of the rats before the administration and 0.25 hour, 0.5 hour, 1 hour, 2 hours, 3 hours, 4 hours, 6 hours, 8 hours, and 12 hours after the administration. Thereafter, amounts of silybin in the obtained blood were measured by using liquid chromatography-mass spectrometry (LC-MS). Here, an in-vivo absorption rate of the total amount of silymarin was indirectly evaluated by measuring amounts of silybin that may be easily measured, instead of the total amount of silymarin. Also, as used herein, the term 'blank' refers to silymarin (i.e., silybin) itself (i.e., no additive).

[Table 5]

| | Amount of silybin in blood (ng/ml) | | | | | | | | | |
|---|---|---|---|---|---|---|---|---|---|---|
| | 0 hr | 0.25 hr | 0.5 hr | 1 hr | 2 hr | 3 hr | 4 hr | 6 hr | 8 hr | 12 hr |
| Liquid additive prepared in Preparation Example 1 | 0.0 | 1969.3 | 918.7 | 753.2 | 659.5 | 185.1 | 242.0 | 22.9 | 19.9 | 12.4 |
| Legalon capsule** | 0.0 | 37.2 | 103.2 | 114.6 | 56.5 | 27.7 | 21.0 | 8.0 | 8.0 | 4.5 |
| Blank | 0.0 | 56.0 | 59.1 | 33.5 | 31.6 | 21.4 | 13.5 | 5.7 | 4.3 | 0.8 |
| ** Legalon Capsule: Legalon Cap. 140 available from Bukwang Pharmaceutical | | | | | | | | | | |

Referring to Table 5 and FIG. 1, an in-vivo absorption rate of the liquid additive prepared in Preparation Example 1 in the rats was higher than those of the commercial liquid additive or silymarin itself.

Evaluation Example 3: Solubility evaluation of silymarin itself with respect to mother liquor

Two types of silymarin were each added to a 50 mL-tube containing 40 ml of whole milk (including solid at an amount of 12 wt%, available from Maeil Dairies Co., Ltd.), fermented milk (Bulgaris Apple, available from Namyang Dairy Product Co., Ltd.), or soymilk (Vegemil A, available from Dr. Chung's Food Co., Ltd.). An adding amount of each of the silymarin was 5 g per 40 ml of whole milk, fermented milk, or soymilk. Also, the liquid additive prepared in Preparation Example 1 was added to a 50 mL-tube containing 30 ml of whole milk (including solid at an amount of 12 wt%, available from Maeil Dairies Co., Ltd.) or fermented milk (Vulgaris Apple, available from Namyang Dairy Product Co., Ltd.). An amount of each of the liquid additives was about 15 g per 30 ml of whole milk or fermented milk. Then, the tubes were each centrifuged by using a centrifuge at a stirring rate of 10,000 rpm for 15 minutes. Subsequently, a supernatant obtained therefrom was filtered through a 0.45 $\mu$m filter to obtain a filtrate. Next, the filtrate was analyzed by high performance liquid chromatography (HPLC) to evaluate a solubility of silymarin itself with respect to the whole milk, fermented milk, or soymilk, and the results are shown in Table 6. In Table 6, the term 'solubility' refers to data of the case when silymarin itself was added to the mother liquor (whole milk, fermented milk, or soymilk), and the term 'maximum solubility' refers to data of the case when the liquid additive prepared in Preparation Example 1 and contains silymarin, was added to the mother liquor (whole milk or fermented milk). Here, the amounts of silymarin in the filtrate were analyzed by using 3-68 silymarin analysis condition described in "2013 Standard and Regulation on Inspection Method of Health Functional Food".

[Table 6]

| Mother liquor | Manufacturer of silymarin | Solubility (mg/mL) | Maximum solubility (mg/mL) |
|---|---|---|---|
| Whole milk | Naturex | 0.32 | 12.29 |
| | Monteloeder | 0.36 | |
| Fermented milk | Naturex | 0.00 | 14.54 |
| | Monteloeder | 0.00 | |
| Soymilk | Naturex | 0.19 | - |
| | Monteloeder | 0.18 | |

Referring to Table 6, when each type of silymarin was added alone, not in the state as the liquid additive prepared in Preparation Example 1, to each of the mother liquors, solubility with respect to the mother liquor was low. Thus, when a beverage is prepared by adding each type of silymarin alone in the mother liquor, an in-vivo absorption rate of the beverage in the body is low, and thus a bioavailability may be low.

Also, referring to Table 6, when a liquid additive containing silymarin was added to whole milk or fermented milk, a solubility of silymarin in the final beverage was about 30 to 40 times higher than that in the case when silymarin itself is added to whole milk or fermented milk. In this regard, it may be easily expected that bioavailability may increase since an in-vivo absorption rate of silymarin in the case when the liquid additive prepared in Preparation Example 1 in whole milk or fermented milk is higher than the case when silymarin itself is added to whole milk or fermented milk.

Evaluation Example 4: Solubility evaluation of liquid additive in functional beverage

40 ml of each of the liquid additive prepared in Preparation Example 1, the functional whole milk prepared in Example 1, and the functional fermented milk prepared in Example 4 was added to a 50 mL-tube. Then, the tubes were left out for a predetermined time period (3 days or 14 days) and centrifuged by using a centrifuge at a stirring rate of 10,000 rpm for 15 minutes. Subsequently, a supernatant was obtained therefrom, and the supernatant was filtered through a 0.45 $\mu$m filter to obtain a filtrate. Next, the filtrate was analyzed by HPLC to measure a remaining amount of silymarin in the filtrate, and the results are shown in Table 7. Here, an increased amount of silymarin in the filtrate denotes a high solubility of silymarin contained in the liquid additive with respect to the functional whole milk or the functional fermented milk. Also, as used herein, the remaining amounts of silymarin, in the filtrate were analyzed by using 3-68 silymarin analysis condition described in "2013 Standard and Regulation on Inspection Method of Health Functional Food"

[Table 7]

| | | Liquid additive of Preparation Example 1 | Functional whole milk of Example 1 | Functional fermented milk of Example 4 |
|---|---|---|---|---|
| Remaining amount of silymarin (wt%*1) | After 3 days | 97.6 | 102.4 | 96.9 |
| | After 14 days | 97.6 | 84.1 | 99.0 |
| *1: | (An amount of silymarin in the filtrate (a measured value))/(an amount of silymarin in the liquid additive (an actual value)) * 100 | | | |

EP 3 090 635 A1

Referring to Table 7, it may be known that silymarin is well dissolved at the same level of the liquid additive prepared in Preparation Example1 in the functional whole milk prepared in Example 1 and the functional fermented milk prepared in Example 4. A range of measurement error acceptable in the art is ±10%, and this explains that a remaining amount of silymarin is over 100%. Also, in the case of the functional whole milk of Example 1, it is deemed that a remaining amount of silymarin after 14 days greatly decreased to 84.1 wt% was because some of the components of silymarin were decomposed. Further, a solubility of silymarin in the functional fermented milk prepared in Example 4 did not have much change over an elapsed time.

Evaluation Example 5: Emulsion formation and size of emulsion evaluation

1 g of the liquid additive prepared in Preparation Example 1 was added to 200 mL of each of 3 types of dispersion media shown in Table 7 and mixed therein. As a result, a first mixture was obtained. As a result of observation with the naked eyes, it was confirmed that an emulsion of the liquid additive dispersed in each of the dispersion media was confirmed that emulsion of the liquid additive dispersed in each of the dispersion media was formed in the first mixture. Then, the first mixture was diluted 1,000 times (in weight) with water to obtain a diluted solution. Then, a size of the emulsion (i.e., a size of a droplet of the liquid additive) in the diluted solution was measured by using Zetasizer Nano ZS (available from Malvern Instrument, U.K.). The measurement was repeated 3 times with respect to the same diluted solution sample, and an average value taken therefrom is shown in Table 8. Here, a buffer solution having a pH of 1.2 is an aqueous hydrochloric acid solution, and a buffer solution having a pH of 6.8 is an aqueous phosphoric acid solution.

[Table 8]

| Dispersion medium | Water | Buffer solution having a pH of 1.2 | Buffer solution having a pH of 6.8 |
|---|---|---|---|
| Size of emulsion (nm) | 188.1± 12.3 | 218.3±53.4 | 303.7±73.3 |

Referring to Table 8, it appeared that the liquid additive prepared in Preparation Example 1 formed an emulsion in a nanometer-size in water, the buffer solution (having pH of 1.2) of the gastric juice condition, or the buffer solution (having pH of 6.8) of the small intestinal juice condition. In this regard, it may be easily expected that a bioavailability of the liquid additive may be high, when the liquid additive is orally administrated, due to its high in-vivo absorption rate. When a size of an emulsion is small, the liquid additive may be effectively absorbed at the gastro-intestinal (GI) tract lining and may be structurally stable at the same time, which may result in an increase in a bioavailability. In light of this result, it may be indirectly confirmed that the functional beverage according to an embodiment of the inventive concept, in which water occupies most portion of the total amount, may also have excellent in-vivo absorption rate and bioavailability.

While the inventive concept has been particularly shown and described with reference to exemplary embodiments thereof, it will be understood that various changes in form and details may be made therein without departing from the spirit and scope of the following claims.

**Claims**

1. A functional beverage comprising:

   a mother liquor; and
   a liquid additive,
   wherein the liquid additive comprises a poorly water-soluble natural product, an edible surfactant, an edible cosurfactant, and an edible oil.

2. The functional beverage of claim 1, wherein the mother liquor is whole milk, coffee milk, soy milk, fermented milk, low-fat milk, fat-free milk, functional milk, or recombined milk.

3. The functional beverage of claim 1, wherein the liquid additive exists in astate of emulsion that is dispersed in the mother liquor.

4. The functional beverage of claim 1, wherein an amount of the liquid additive is in a range of 0.5 parts to 10 parts by weight based on 100 parts by weight of the mother liquor.

5. The functional beverage of claim 1, wherein the liquid additive comprises 100 parts by weight of the poorly water-soluble natural product, about 300 parts to about 8,000 parts by weight of the edible surfactant, about 40 parts to about 2,500 parts by weight of the edible cosurfactant, and about 40 parts to about 1,500 parts by weight of the edible oil.

6. The functional beverage of claim 1, wherein the poorly water-soluble natural product comprises at least one type of a poorly water-soluble natural polyphenol compound selected from curcumin, silymarin, and resveratol.

7. The functional beverage of claim 1, wherein the edible surfactant comprises polysorbates.

8. The functional beverage of claim 1, wherein the edible cosurfactant comprises at least one selected from glycerol fatty acid esters, propylene glycols, propyleneglycol fatty acid esters, and medium chain triglycerides.

9. The functional beverage of claim 1, wherein the edible oil comprises at least one selected from vegetable oil, refined fish oil, and oils derived from seaweed.

10. The functional beverage of claim 5, wherein the liquid additive further comprises an edible additive at a ratio of about 500 parts by weight or lower.

11. The functional beverage of claim 10, wherein the edible additive comprises at least one compound selected from lecithin, a viscosity controlling agent, flavoring agent, preservatives, colorant, glycerol, sorbitol, and gelatin.

# FIG. 1

## INTERNATIONAL SEARCH REPORT

International application No.

**PCT/KR2014/006253**

### A. CLASSIFICATION OF SUBJECT MATTER

*A23L 2/38(2006.01)i, A23C 9/00(2006.01)i, A23L 2/52(2006.01)i*

According to International Patent Classification (IPC) or to both national classification and IPC

### B. FIELDS SEARCHED

Minimum documentation searched (classification system followed by classification symbols)
A23L 2/38; A61K 9/10; A61K 9/107; A61K 38/13; A61K 31/357; A23C 9/00; A23L 2/52

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: mother liquid, liquid additive, poorly water-soluble natural product, edible surfactant, edible cosurfactant, edible oil, functional beverage, milk

### C. DOCUMENTS CONSIDERED TO BE RELEVANT

| Category* | Citation of document, with indication, where appropriate, of the relevant passages | Relevant to claim No. |
|---|---|---|
| X<br>A | KR 10-2005-0075074 A (KOREA UNITED PHARM. INC.) 20 July 2005<br>See abstract; examples 1-5; table 1; claims 1-7. | 1,3,5-11<br>2,4 |
| X<br>A | KR 10-0494096 B1 (HANMI PHARM. CO., LTD.) 13 June 2005<br>See abstract; page 2 line 44 - page 3 line 33; claims 1, 2, 4, 5. | 1,3,5,7-11<br>2,4,6 |
| A | KR 10-0167696 B1 (NOVARTIS AG) 15 January 1999<br>See abstract; claim 1. | 1-11 |
| A | KR 10-0507771 B1 (HANMI SCIENCE CO., LTD) 17 August 2005<br>See abstract; claims 1, 2, 4-6. | 1-11 |
| A | KR 10-2005-0029965 A (KONGJU NATIONAL UNIVERSITY INDUSTRY-UNIVERSITY COOPERATION FOUNDATION) 29 March 2005<br>See abstract. | 1-11 |

| ☐ | Further documents are listed in the continuation of Box C. | ☒ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 OCTOBER 2014 (24.10.2014) | **27 OCTOBER 2014 (27.10.2014)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

INTERNATIONAL SEARCH REPORT
Information on patent family members

International application No.

**PCT/KR2014/006253**

| Patent document cited in search report | Publication date | Patent family member | Publication date |
|---|---|---|---|
| KR 10-2005-0075074 A | 20/07/2005 | KR 10-0588771 B1 | 14/06/2006 |
| KR 10-0494096 B1 | 13/06/2005 | KR 10-2004-0013286 A | 14/02/2004 |
| KR 10-0167696 B1 | 15/01/1999 | CN 1104256 C | 02/04/2003 |
| | | CN 1128671 A | 14/08/1996 |
| | | EP 0711550 A1 | 15/05/1996 |
| | | EP 0711550 B1 | 16/01/2002 |
| | | JP 3391961 B2 | 31/03/2003 |
| | | KR 10-1996-0016904 A | 17/06/1996 |
| | | US 5603951 A | 18/02/1997 |
| KR 10-0507771 B1 | 17/08/2005 | AU 2004-277710 A1 | 07/06/2004 |
| | | CN 100463670 C | 25/02/2009 |
| | | CN 1735401 A | 15/02/2006 |
| | | EP 1558214 A1 | 03/08/2005 |
| | | EP 1558214 A4 | 09/12/2009 |
| | | EP 1558214 B1 | 12/09/2012 |
| | | JP 2006-508104 A | 09/03/2006 |
| | | KR 10-2004-0040882 A | 13/05/2004 |
| | | US 2006-0062810 A1 | 23/03/2006 |
| | | WO 2004-041249 A1 | 21/05/2004 |
| KR 10-2005-0029965 A | 29/03/2005 | KR 10-0562460 B1 | 22/03/2006 |

Form PCT/ISA/210 (patent family annex) (July 2009)